# EUROPEAN PATENT APPLICATION

(11) **EP 2 042 154 A1**
(43) Date of publication of application: **01.04.2009**
(21) Application number: 07117472.6
(22) Date of filing: 28.09.2007
(51) Int. Cl.: A61K 8/60, A61Q 19/00

(54) **Cosmetic mousse**

(71) Applicant: Koninklijke Coöperatie Cosun U.A., 4814 ND Breda (NL)
(72) Inventor: Blauwhoed, Johannes Petrus Jacobus, 4731 GL, Oudenbosch (NL); Wouters, Christel Louise Jozef, BE-2547, Lint (BE)
(74) Representative: Swinkels, Bart Willem

(57) **Abstract**

The invention pertains to An aerated cosmetic mousse, being an oil-in-water emulsion containing: 0.5 - 5 wt% of at least one sucrose ester; 05 - 5 wt% of a gelling agent; 10 - 50 wt% fat, provided that 10 - 20 wt% is solid at room temperature; and water, wherein all weight percentages are calculated on the total weight of the above. The mousse incorporates 20 - 80 vol% air, based on the total mousse volume, and contains less than 0.3 wt% of fatty acids having C6-C22 alkyl incorporated in the mousse. The mousse is stable at room temperature for at least 30 days, and heat stable at 40 °C for more than two weeks.

## Description

### FIELD OF THE INVENTION

The invention is in the field of cosmetics, and in particularly pertains to an aerated cosmetic mousse.

### BACKGROUND OF THE INVENTION

Natural and food-grade ingredients become more and more popular within cosmetics. Related thereto, there is a lot of interest in mousse-like structures, especially with the appearance of a chocolate mousse. While it is accepted in food applications that such products only have a limited shelf-life, a cosmetic mousse must meet a long term stability (up to 3 years) and heat stability (3 months at 40°C). In addition, the product must have a shockproof stability, good spreading properties and nice skin feel. These are very challenging problems, especially related to the air bubble stabilization, and prevented easy implementation of mousses widely spread in food applications into the world of cosmetics so far.

WO-A-2005/117813, on behalf of l'Oreal, relates to mousse or soufflé type cosmetic products. To create a springy, luxuriant feel, and excellent spreading and covering properties, the product taught therein is based on an anhydrous mixture of silicone elastomers, a non-aqueous or anhydrous silicone-based solvent and a water-insoluble structuring agent. However, the term "mousse" as used in WO-A-2005/117813, and as it is presently applied in the field, is misleading. It involves powdery mixtures in which air hardly plays any role. Despite these efforts, any attempt with powdery mixtures to mimic an aerated mousse, such as a chocolate mousse, is deemed to failure.

On the other hand, US-A-2006/039936, also in the name of l'Oreal, discloses an emulsification system for use in cosmetics, wherein the composition comprises a mixture of specific high HLB and low HLB surfactants, including sucrose fatty acids esters of vegetable origin, to emulsify a mixture of polar and non-polar oils. It may further contain up to 5 wt% gelling agents. These types of emulsions are intended for applying make-up to keratinous tissue, and applications typically involve lotions or fluid creams. Hence, US-A-2006/039936 does not disclose a mousse-like composition.

An aerated cosmetic composition is indeed disclosed in FR-A-2.850.017. The o/w composition allegedly incorporates large amounts of gas, stabilized by a combination of sugar derivatives and less than 10 wt% fatty acids having 6 to 22 carbon atoms, of the total weight of the formulation. Throughout the examples, soaps are formed from the fatty acids by adding alkaline materials such as sodium or potassium hydroxide, triethanolamine or aminomethylpropanediol to the composition. The "saponified" fatty acids, together with the sucrose derivates, preferably alkylglucosides, function as emulsifiers.

The composition according to FR-A-2.850.017 should overcome all kinds of problems, such as skin irritation, which are normally associated with large amounts of fatty acids conventionally applied in the art. Still, it teaches to use significant amounts of more than 1 % of fatty acids. In fact, it is reported that good stability is reached with a minimum level of fatty acids of 2.5 wt%. The necessity of fatty acids in the composition of FR-A-2.850.017 is indeed confirmed here, in the accompanying examples. The composition further comprises 0.05 - 1 wt% of hydrocolloids and 0.1 - 2 wt% gelling agents. No further details on specific hydrocolloid compositions are given. Sodium alginate appears to be the preferred hydrocolloid/gelling agent.

It was the inventors' findings that compositions within the ranges claimed in FR-A-2.850.017 behaved overall poorly. Observations such as insufficient aeration, instabilities, lack of spreadability were common. Compositions were obtained that could be cut into pieces, which inevitably makes them useless for mousse or cream applications. The results of the tests are incorporated in the accompanying examples.

Hence, there is a need to further reduce the amounts of fatty acids in the composition, and to provide an aerated cosmetic mousse with a durable and heat-stable "chocolate mousse" appearance, excellent spreading properties and good skin-feel, without irritating the skin upon use.

### SUMMARY OF THE INVENTION

It is an object of the invention to provide a stable aerated cosmetic mousse which fulfils the requirements with respect to structure, (heat) stability, skin feel and spreading properties for cosmetic use, having a "chocolate mousse"-like appearance, and without the disadvantages reported in the art.

The inventors have found that a mousse can be produced using a specific combination of emulsifiers, gelling agents, and solid fat, thereby keeping the amounts of fatty acids at a minimum. In fact, it was found that it is no longer necessary to deliberately add fatty acids to the composition.

Thereto, one or more sucrose esters were selected as an emulsifying agent, stabilizing an oil-in-water emulsion. Advantageously, the class of sucrose esters has recently been certified as natural-grade according to cosmetics classifications. The world-wide need for natural products makes such a "natural-grade" cosmetic mousse even more acceptable. The amount of fatty acids in the composition can be as low as zero. However, for practical reasons trace amounts of fatty acids is allowed for. Fatty acids may sometimes be found in fats and the sucrose esters which are applied in the cosmetic mousse of the invention.

Moreover, the combination of a range of gelling agents and significant amounts of solid fat also contribute to the required firmness, foamability and durability.

The amount of solid fat present in the w/o emulsion should be at least 10 wt%, much larger than the combinations of wax and vegetable fat used in the examples of FR-A-2.850.017. For sake of completeness, it is emphasized that fat is not incorporated in the mousse to replace the fatty acids mentioned therein. Where fatty acids have been applied as emulsifiers up to present, the fat obviously does not contribute to the emulsification of the composition. More details are provided below.

Due to its unmatched properties, the cosmetic mousse of the invention may find use in a new line of cosmetic products, where skin feel and spreadability form key issues, or it may replace or improve presently employed spread systems, for instance in sunscreens, creams, foundations, eye shadows and blushes.

### DETAILED DESCRIPTION OF THE INVENTION

The invention thus pertains to an aerated cosmetic mousse, being an oil-in-water (o/w) emulsion containing:
(a) 0.5 - 5 wt% of at least one sucrose ester;
(b) 0.05 - 5 wt% of a gelling agent, preferably gellan gum, methylcellulose and/or modified starch;
(c) 10 - 50 wt% fat, provided that 10 - 20 wt% is solid at room temperature; and
(d) water,
   wherein the weight percentages are calculated on the total weight of (a) - (d), and wherein said mousse incorporates 20 - 80 vol% air, based on the total mousse volume,
   wherein the amount of fatty acids incorporated in the mousse is less than 0.3 wt%, based on the total weight of the composition,
   said mousse being stable at room temperature for at least 30 days.

The mousse is an oil-in-water emulsion which remains stable for prolonged periods of time. The cosmetic mousse thus distinguishes from food-related mousses with limited shelf life, if at all at room temperature. In food industry, mousses are prepared using gelatine for the stabilizing agent. However, gelatine has no satisfactory stabilising properties at room temperature. A cosmetic mousse is preferably free from gelatine. The shelf-life of the mousse of the invention is typically more than 0.5 year, more preferably more than 1 year, most preferably more than 2 years at normal storage conditions at room temperature.

The term "mousse" as used herein is understood to mean an aerated and spreadable composition containing small air bubbles trapped in a matrix, otherwise referred to as a cosmetic base. It may be completed with active ingredients depending on the intended use. The spreadability implies that the composition exhibits gel-like rheological behavior at room temperature. For practical purposes, the mousse shows a transition to liquid-like behavior at a temperature higher than 40 °C.

Its hardness, as measured with a Texture Analyser, is preferably of the order of 20 - 200 g, more preferably 80 - 160 gram. Details are given in the accompanying

### examples.

Additionally, in the field of cosmetics the mousse should be heat-stable at 40 °C for a period of time of more than 30 days, preferably more than 2 months, more preferably more than 3 months.

Although the cosmetic use is broadly defined, hair care compositions, such as hair foams, are not part of the invention. The mousse's favorable properties, including excellent skin-feel, are especially directed to skin-applied cosmetic products.

Throughout the text, the terms "aerated" and "foamed" are considered interchangeable. The degree of aeration may be characterized by the density of the aerated mousse divided by the density of the unaerated composition, multiplied by 100 %. The air is incorporated in the mousse structure as small air pockets, giving it its favorable chocolate-mousse appearance. In practice the above percentage can be calculated by determining the density of the mousse before and after heating. By heating, the air is removed from the mousse and a measure for the unaerated mousse can be obtained.

Alternatively, the mousse preferably incorporates 20 - 80 vol%, more preferably 30 - 70 vol% and most preferably at least 50 vol% air, based on the total volume of the mousse.

It is preferred that (a) - (d) make up for at least 85 wt%, more preferably at least 90 wt% of the mousse, the remainder being largely formed from active ingredients. Additionally, water forms preferably between 65 - 80 wt% of the total weight of the mousse. The actual amounts of the other ingredients (a) - (c) in the total composition may be calculated there from.

As mentioned before, the cosmetic mousse of the invention may be prepared without deliberately adding any amount of fatty acids. Fatty acids are preferably present in trace amounts only, as a contaminant introduced with the other ingredients, e.g. related with the sucrose ester synthesis. The fatty acids do not contribute to the invention, in contrast with the teachings of FR-A-2.850.017. The amounts of fatty acids is preferably less than 0.2 wt%, more preferably less than 0.1 wt%, based on the total weight of the composition. Fatty acids are understood to comprise alkyl chains having a carboxylic end group, preferably C6 - C22 alkyl, more preferably those having at least C16 alkyl. Since these fatty acids do not contribute to the composition, there is no need for saponifying these fatty acids either. Hence, if fatty acids are present in the composition at all, they are unprocessed, meaning "unsaponified". In view thereof, there is no need to add any alkaline materials to the composition either. If at all, less than 1 wt%, more preferably less than 0.2 wt% of alkaline material, such as hydroxide and/or amine salts, in particular potassium or sodium hydroxide, triethanolamine and/or aminomethylpropanediol, is included The weight percentages are based on the weight of the total composition.

The essential components of the mousse are now discussed individually.

### Sucrose ester

Although not limited thereto, the composition comprises at least one sucrose ester, which is a non-ionic emulsifier, generally recognized as "mild" in the cosmetic field. Furthermore, it has been tested dermatologically-acceptable in the past. The amount sucrose esters(s) is effective to emulsify the mousse. In general, the amount ranges from 0.5 - 5 wt%, more preferably 1 - 4 wt%, most preferably up to 3 wt%, based on the total weight of (a) - (d). The specified amount is based on the total amount of sucrose esters present.

The sucrose ester is preferably sucrose esterified with fatty acids having C16 - C20 alkyl chains, preferably palmitic acid and/or stearic acid, preferably a combination of these fatty acids. Both sucrose monoesters and sucrose diesters may be applied, wherein sucrose diesters may comprise identical or different fatty acid chains.

The sucrose esters used in the composition preferably have a "weight-averaged HLB value" of 9 - 13, preferably 10 - 12. The HLB value(s) is/are determined experimentally by emulsifying mineral oil, having a known required HLB, with a combination of sucrose ester and another emulsifier of which the HLB is known.

Furthermore, it is preferred that all incorporated sucrose esters independently have an HLB value within the range of 5 - 16. Preferred sucrose esters comprise sucrose monostearate and distearate. Suitable candidates are commercially available with Sisterna, such as SP30-C, SP50-C and SP70-C, having HLB values according to their product specifications of 6, 11 and 15, respectively. Therein, the number "30", "50" and "70" corresponds to the percentage of monoester. SP50-C is a favored sucrose ester, since its HLB value makes it suitable without further aid; however, combinations of SP30-C and SP70-C, preferably in weight ratios ranging from 2:1 - 1: 2 are equally attractive.

### Gelling agent

The mousse contains at least one gelling agent that is able to gelify at room temperature. The amount thereof deemed necessary to induce gelling varies with the gelling agent of choice: gellan gum and/or methylcellulose may be applied in smaller amounts than modified starch, and combinations may exhibit synergistic effects. It will readily occur to the skilled person how to select a suitable gelling agent, including the amount thereof. As a guidance: good results are obtained with 0.05 - 1 methyl cellulose and gellan gum independently, and 1 - 4 wt% modified starch.

Although alginate is such a suitable heat-stable thickening agent, it is not the preferred choice: Alginate gelifies only upon addition of calcium ions. The timing of calcium-induced gelation may hinder mousse formation: early gelation makes it impossible to aerate the formulation, while late gelation affects mousse stability negatively. The inventors have found excellent gelling properties when using at least one of gellan gum, methylcellulose or modified starch, or combinations thereof.

Most preferably, at least gellan gum and/or modified starch is present.

It is preferred that the above thickening agents are present in an amount of 0.3 - 3 wt%, more preferably 0.4 - 2 wt% of the total weight of (a)-(d). If other thickening agents are present, these are not considered to add to the weight of class b). However, the invention does not exclude the use of other hydrocolloids in the composition.

Optionally, the mousse may further contains hydrocolloids other than gelling agents for reasons of stabilizing and thickening the composition. Typical examples are xanthan gum, microcrystalline cellulose. However, these hydrocolloids are not accounted for in fraction b) of the mousse. These are typically present in an amount of 0.05 - 3 wt% of the total formulation.

### Fat

Although the term "fat" is often used in the art to characterize fats which are in solid form at room temperature, in the context of the invention the terms "fat" and "oil" are considered interchangeable. Both fats and oils may be applied, provided that the melting behavior of the mousse fulfils the requirements of the invention. The fat fraction also incorporates waxes, butter, phospholipids, vegetable fats and fatty alcohols.

The amount of fat in the mousse is relatively large, preferably 15 - 40 wt%, more preferably 20 - 30 wt% calculated over a)-d). Independently, in order to create a firm mousse-like structure, it is preferred that the fat fraction comprises a large amount of fats which are solid at room temperature, preferably 10 - 20 wt%, more preferably 12-18 wt%, calculated on the total amount of components a)-d). At too low concentrations, instability issues play a role. Higher concentrations reduce the spreadability.

Suitable candidates for incorporation in the fat fraction are vegetable oils, all or not hydrogenated, including butters and waxes, e.g. hydrogenated palm or rapeseed oil, cacao or shea butter, and vegetable waxes like carnaubawax or bees wax. Obviously, all kinds of cosmetic oil phase ingredients as presently applied are equally useful.

Given the aforementioned numbers on total fat and the portion of solid fat therein, it is thus possible that fraction c) consists of solid fat only. However, the invention also encompasses the use of additional non-solid fats. However, for cost reasons, the amounts thereof are typically maintained as low as possible.

Typically, the mousse may contain small amounts of preservatives, or mixtures thereof. A typical cosmetic ingredient is a moisturizing agent, such as glycerin or sorbitol.

The mousse of the present invention further comprises suitable (e.g., pharmaceutically, cosmetically or dermatologically acceptable) active agents, including, for example, coloring agents (e.g., pigments and dyestuffs), antioxidants, essential oils, preserving agents, fragrances, fillers, neutralizing agents, UV filters, topical antibiotics or steroids, and polymers, e.g., lipo-soluble polymers. These active ingredients are most notably topically active or transdermally deliverable pharmaceuticals. Examples of dermatological active agents e.g., agents capable of treating or preventing any sign of ageing of the skin. The active agents may be chosen, for example, from moisturizers, free-radical scavengers, keratolytic agents, vitamins, anti-elastase and anti-collagenase agents, peptides, steroids, trace elements, bleaching agents, extracts of algae and of planktons, sunscreens, enzymes and coenzymes, flavonoids and ceramides, alpha-hydroxy acids and mixtures thereof. The skilled person is well familiar with such active ingredients and desired amounts thereof, especially those applied in personal care products for which the mousse of the invention may form an improvement.

The mousse is efficiently processable and can be packaged in a suitable container or other receptacle where it can maintain a shape with little or no flow after packaging. The choice of container may depend upon the intended purpose of the mousse.

The invention also pertains to a process for manufacturing the aerated mousse suitable for cosmetic use according to the present invention, involving mixing the ingredients and any additives and adjuvants together, thus forming and stabilizing an o/w emulsion, and aerating the mixture, to obtain an aerated or foamed mousse.

There is no particular order in mixing, provided that the air is to be incorporated after emulsification. Usually, mixing and emulsification is realized at elevated temperatures, typically in the range of 45 - 90 °C.

Some active ingredients are heat-sensitive. Thus, in such case it is preferred to incorporate these active ingredients only after some intermediate cooling, at a temperature below 35 °C.

The incorporation of air can be established by regular whipping and mixing as traditionally applied in for food-related applications, such as chocolate mousse. Such mechanical action may be performed at the elevated temperature handled during preceding emulsification, but it is preferred to cool the formulation to be aerated down to a temperature below 35 °C.

### EXAMPLES

### Example 1 - Cosmetic formulation

A formulation is prepared from the ingredients and amounts thereof listed in table 1. The numbers are based on the total weight of the formulation.

| | Ingredients | Source | wt%* | wt%* |
|---|---|---|---|---|
| (a) | sucrose stearate | Sisterna SP70-C | 1.0 | 1.1 |
| | sucrose distearate | Sisterna SP30-C | 1.0 | 1.1 |
| | | | | |
| (b) | gellan gum | Kelcogel CG-HA | 0.15 | 0.16 |
| | methylcellulose | Methocel A4M | 0.10 | 0.11 |
| | | | | |
| (c) | Hydrogenated palm fat | | 14 | 15 |
| | sunflower oil | | 4.0 | 4.3 |
| | | | | |
| (d) | water | | 72 | 78 |
| | | | | |
| | microcrystalline cellulose | Avivell PC611 | 0.5 | 0.5 |
| | xanthan gum | Keltrol CG-SFT | 0.2 | 0.2 |
| | | | | |
| | glycerine | | 5 | 5 |
| | preservative | Euxyl k100 | 0.2 | 0.2 |

| | | | | |
|---|---|---|---|---|
| * Based on the total weight of the formulation ** Recalculated, based on the total weight of (a) - (d) | | | | |

The w/o emulsion thus obtained was then aerated at a temperature of 20 °C using a Hobart mixer, to obtain a mousse having 60 vol% of air incorporated therein.

The firmness of the aerated composition was tested using texture analysis (with a QTS25 Stevens Texture Analyser, settings: Probe TA3, speed 30 mm/min, distance 10 mm). A hardness of about 120 gram was found.

### Comparitive example - Test results on FR-A-2.850.017

O/w compositions were prepared on the basis of the information provided in FR-A-2.850.017, selecting sucrose ester as the sugar derivative.

Tests involved the ingredients and amounts thereof as given in claims 11 and 12 of the French patent application, prepared according to the recipe given in its description. Where general terms are given, a selection was made in line with the preferred embodiments disclosed in FR-A-2.850.017, and as exemplified. Therein, the compositions were tested using the alkylglucosides (montanov® 68) and the sucrose esters SP70 mentioned in FR-A-2.850.017.

To obtain reliable results, a number of experiments was conducted at the upper and lower limits of the claimed ranges and in the middle of these ranges, where the skilled person is to expect optimum results. For sake of completeness, in experiment 4 the effect of fatty acids only was tested (leaving out sugar derivates), and in experiments 5 and 6 the fatty acids were left out of the composition. It is noted that these three experimental settings do not represent the disclosure of FR-A-2.850.017, which teaches to minimize but not to avoid fatty acids.

The o/w compositions thus obtained were then aerated, and the uptake of gas was characterized by the density. Realizing that an unaerated sample exhibited a density of about 1 g/ml, a density of 0.46 corresponds to 54 % gas uptake.

The results were summarized in the table below. Emphasis was added to the components which play an important role as an emulsifier in the composition.

If fatty acids were not incorporated into the composition, less than 20 % of aeration was achieved. Hence, experiments 5 and 6 show that the presence of fatty acids are a necessity in the compositions taught in FR-A-2.850.017. Without those, aeration is negligible. On the other hand, only fatty acids were found not to be sufficient either, as shown in experiment 4. Hence, from those experiments it is safe to conclude that the compositions of FR-A-2.850.017demand a combination of sugar derivatives and the fatty acids.

The remainder of the experiments were based on significant amounts of both. All cases showed significant uptake of gas. Still, the results were poor in other aspects: The composition obtained in experiment 2 was very viscous and exhibited a dough-like structure, while a very instable gel-like structure was observed in experiment 3: the structure collapsed already when stored at room temperature for few days only. No stable and significantly aerated structure was obtained. If stressed, the structure crumbled. If the firmness of the composition was tested using texture analysis (with a QTS25 Stevens Texture Analyser, settings: Probe TA3, speed 30 mm/min, distance 10 mm), a hardness far outside the desired range of 20 - 200 g was found. Moreover, phase separation occurred in case of experiment 4.

Although respectable amounts of gas could be aerated into a mousse prepared according to experiment 1, still it lacked stability over a period of time deemed necessary for a cosmetic mousse. Within 2 weeks, air disappeared from the sample, and the mousse structure collapses. Hence, no satisfactory heat stability was achieved.

Based thereon, it is concluded that the information given in FR-A-2.850.017 do not enable the skilled person to reproduce a stable, aerated mousse-like structure with more than 20 % gas as it is claimed.

**Table: Test results on aerated w/o compositions according to FR-A-2.850.017**

| | Claim 11 in FR-A-2.850.017 | exp 1 | exp 2 | exp 3 | exp 4 | exp 5 | exp 6 |
|---|---|---|---|---|---|---|---|
| A | | | | | | | |
| water | 50-80 | 74 | 51.05 | 79.65 | 76 | 77 | 77.5 |
| glycerol | 1-20 | 5 | 5 | 5 | 5 | 5 | 5 |
| EDTA | 0.05 - 0.3 | 0.1 | 0.1 | 0.1 | 0,1 | 0.1 | 0.1 |
| xantan gum*** | 0.1 - 2 | 0.15 | 2 | 0.1 | 0.15 | 0.15 | 0.15 |

| B | | | | | | | |
|---|---|---|---|---|---|---|---|
| **stearic acid** | **1 -10** | **3** | **3** | **1** | **3** | | |
| **Sisterna SP70C** | | **2** | | | | **2** | **2** |
| **Montanov® 68** | **0.5-5** | | **3** | **0.5** | | | |
| beeswax* | 0.5-5 | 1.5 | 4 | 1 | 1.5 | 1.5 | 1.5 |
| shea butter* | 0.5 - 10 | 3 | 8 | 1 | 3 | 3 | 3 |
| IPM (isoprpylmiristate)* | 1 - 25 | 8 | 20 | 10 | 8 | 8 | 8 |
| euxyl K300 (preservative) | qs | 1 | 1 | 1 | 1 | 1 | 1 |
| Tocopherol | qs | 0.1 | 0.1 | 0.1 | 0.1 | 0.1 | 0.1 |
| | | | | | | | |

| C | | | | | | | |
|---|---|---|---|---|---|---|---|
| **triethanolamine** *** | **0.2-1.5** | **0.5** | **0.5** | **0.25** | **0.5** | **0.5** | |
| | | | | | | | |

| D | | | | | | | |
|---|---|---|---|---|---|---|---|
| alginate*** | 0.05-1 1 | 0.4 | 1 | 0.1 | 0.4 | 0.4 | 0.4 |
| Tetrasodium | | | | | | | |
| pyrophosphate | 0-2 | 0.6 | 0.6 | 0.1 | 0.6 | 0.6 | 0.6 |
| Calciumsulphate | 0-2 | 0.65 | 0.65 | 0.1 | 0.65 | 0.65 | 0.65 |
| Total | | 100 | 100 | 100 | 100 | 100 | 100 |
| Density (g/ml) | | 0.58 | 0.72 | 0.4 | 0.93 | 0.83 | 0.82 |

| | | | | | | | |
|---|---|---|---|---|---|---|---|
| ** triethanolamine = base alcaline *** xanthan gum and alginate are Gélifant/hydrocolloide | | | | | | | |

## Claims

1. An aerated cosmetic mousse, being an oil-in-water emulsion containing:
(a) 0.5 - 5 wt% of at least one sucrose ester;
(b) 0.05 - 5 wt% of a gelling agent;
(c) 10 - 50 wt% fat, provided that 10 - 20 wt% is solid at room temperature; and
(d) water,
wherein all weight percentages are calculated on the total weight of (a) - (d), wherein said mousse incorporates 20 - 80 vol% air, based on the total mousse volume,
and wherein the amount of fatty acids having C6-C22 alkyl incorporated in the mousse is less than 0.3 wt%, based on the total weight of the composition,
said mousse being stable at room temperature for at least 30 days.

2. The mousse according to claim 1, said gelling agent comprising gellan gum, methylcellulose and/or modified starch.

3. The mousse according to claim 1 or 2, containing less than 0.1 wt% of said fatty acids in the composition.

4. The mousse according to any one of the preceding claims, further containing one or more pharmaceutically, cosmetically or dermatologically acceptable active agents.

5. The mousse according to any one of the preceding claims, wherein said at least one sucrose esters independently have an HLB value within the range of 5 - 16.

6. The mousse according to any one of the preceding claims, wherein said at least one sucrose ester has or have a weight-averaged HLB value of 9 - 13.

7. The mousse according to any one of the preceding claims, wherein said at least one sucrose ester comprises sucrose monostearate and sucrose distearate.

8. The mousse according to any one of the preceding claims, wherein (a)-(d) make up for at least 85 wt% of the total formulation.

9. Use of the aerated cosmetic mousse according to any one of the preceding claims in cosmetics.
